(19) Europäisches Patentamt
European Patent Office
Office européen des brevets

(11) Numéro de publication: **0 074 903**
**B1**

(12) # FASCICULE DE BREVET EUROPÉEN

(45) Date de publication du fascicule du brevet: **05.11.86**

(21) Numéro de dépôt: **82401654.7**

(22) Date de dépôt: **10.09.82**

(51) Int. Cl.[4]: **C 07 C 91/40**, C 07 C 93/14, C 07 C 103/44, A 61 K 31/135, A 61 K 31/16

(54) **Dérivés d'amino-2 tétrahydro-1,2,3,4 naphtalène, leur préparation et leur application en thérapeutique.**

(30) Priorité: **16.09.81 FR 8117454**

(43) Date de publication de la demande: **23.03.83 Bulletin 83/12**

(45) Mention de la délivrance du brevet: **05.11.86 Bulletin 86/45**

(84) Etats contractants désignés: **AT BE CH DE FR GB IT LI LU NL SE**

(56) Documents cité:
**DE-A-2 803 582**
**US-A-4 035 512**

**CHEMICAL ABSTRACTS, vol. 88, no. 15, 10 avril 1978, résumé no. 104974d, page 537 COLUMBUS OHIO (US) & Chem. Pharm. Bull. 1977, 25(12), 3289-300 A. MIYAKE et al.: "The synthesis of N,N'-disubstituted 2,5-diamino-6-hydroxy-1,2,3,4-tetrahydro-1-naphthalenol derivatives"**

**Le dossier contient des informations techniques présentées postérieurement au dépôt de la demande et ne figurant pas dans le présent fascicule.**

(73) Titulaire: **SYNTHELABO, 58, rue de la Glacière, F-75621 Paris Cedex 13 (FR)**

(72) Inventeur: **Beeley, Nigel, 5, bis résidence du Parc, F-91120 Villebon s/Yvette (FR)**
Inventeur: **Cremer, Gérard, 10, rue Maurice Ravel, F-91380 Chilly- Mazarin (FR)**
Inventeur: **Dimsdale, Michael, 5, Rue de la Grenouillette, F-78180 Montigny- le- Bretonneux (FR)**
Inventeur: **Manoury, Philippe, 9, rue de Malabry, F-92350 Le Plessis- Robinson (FR)**

(74) Mandataire: **Ludwig, Jacques, SYNTHELABO Service Brevets 58, rue de la Glacière, F-75621 Paris Cedex 13 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 074 903 B1

LIBER, STOCKHOLM 1986

**Description**

La présente invention a pour objet des dérivés d'amino-2 tétrahydro-1,2,3,4 naphthalène, un procédé pour les préparer, et leur application en thérapeutique.

Des dérivés d'amino-2 tétrahydro-1,2,3,4 naphthalène sont déjà connus selon la demande de brevet DE-A-28 03 582.

Les composés de l'invention répondent à la formule générale (I)

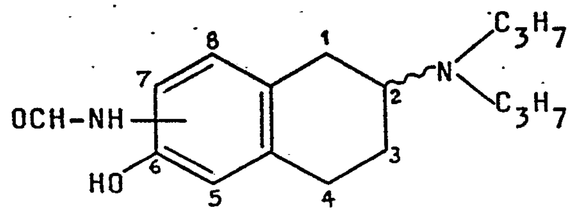

(I)

dans laquelle le groupe -NH-CHO est en ortho du groupe -OH.

Leurs sels d'addition à des acides acceptables en pharmacologie font également partie de l'invention.

Les composés de l'invention comportant dans leur molécule un atome de carbone asymétrique (position 2), ils peuvent se présenter sous forme de racémates ou d'énantiomères.

Les composés de l'invention peuvent être préparés à partir de méthoxy-6 tétrahydro-1,2,3,4 naphtalénone-2, produit du commerce, répondant à la formule (II)

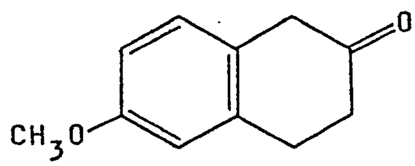

(II)

que l'on fait réagir avec la benzylamine ou avec la dipropylamine de formule (III)

$C_3H_7$-NH-$C_3H_7$ (III)

pour obtenir l'amino-2 méthoxy-6 dihydro-3,4 naphtalène de formule (IV)

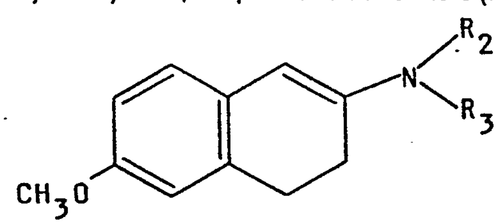

(IV)

dans laquelle -$NR_2R_3$ est le groupe amino ou dipropylamino.

Par hydrogénation sous pression on obtient l'amino-2 tétrahydronaphtalène de formule (V)

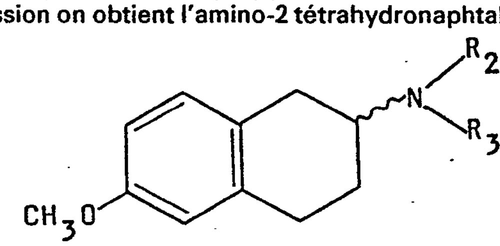

(V)

Le composé (V) dans la formule duquel-$NR_2R_3$ désigne le groupe dipropylamino peut également être obtenu par alkylation du composé (V) correspondant dans la formule duquel -$NR_2R_3$ désigne le groupe amino. L'alkylation peut se faire de manière connue, par exemple sur une suspension d'un sel de l'amine primaire (V), avec un halogénure de propyle approprié, de préférence l'iodure, en présence d'une base telle que le carbonate de potassium. Cette méthode de préparation est à préférer lorsque l'on souhaite préparer un seul énantiomère d'un composé de formule I. Il est en effet plus facile de séparer les énantiomères de l'amine primaire de formule V (-$NR_2R_3$=$NH_2$) que les énantiomères du composé final. On soumet ensuite l'amine (V) à une nitration. Celle-ci fournit un mélange de deux isomères nitréa respectivement en 5 et 7 d'après la formule (VI)

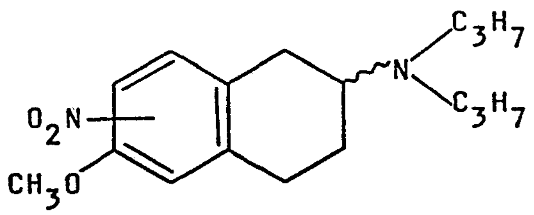

(VI)

Les dérivés de formule (VI) sont nouveaux et font également partie de l'invention. On procède ensuite à la déméthylation de l'un des dérivés nitrés, après l'avoir séparé de son isomère, obtenant ainsi un sel d'hydroxy-6 amino-2 tétrahydronaphtalène de formule (VII)

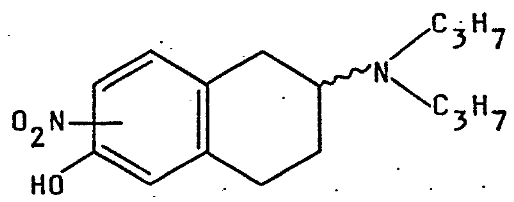

(VII)

Par réduction catalytique (Pd, Ni Raney) du groupe nitro du composé de formule (VII) on aboutit à la diamine formule (VIII)

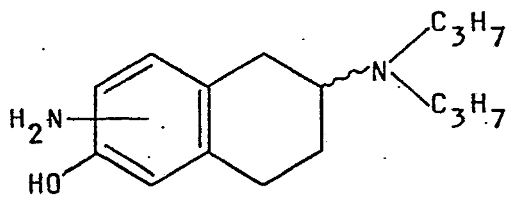

(VIII)

que l'on soumet enfin à une N formylation, pour obtenir le composé de formule (I).

La condensation de la dipropylamine (III) avec la tétrahydronaphtalénone (II) peut se faire à chaud, dans un solvant non polaire tel que le benzène, en présence d'un acide tel que l'acide paratoluènesulfonique. On procède ensuite à une hydrogénation dans le même récipient de réaction en présence d'un catalyseur d'hydrogénation et sous pression.

La nitration de l'amino-2 tétrahydronaphtalène (V) peut se faire de manière connue. Lorsque le groupe amino n'est pas alkylé ($R_2$=H), il convient de protéger préalablement l'azote en l'acylant, par exemple au moyen d'anhydride acétique, et en éliminant de nouveau le groupe acétyle à la fin du processus de préparation.

On sépare ensuite les deux isomères nitréa (VI) de manière connue, par exemple par chromatographie sur colonne.

La déméthylation du dérivé nitré, séparé de son isomère, s'effectue par exemple par action de l'acide bromhydrique ou par action de tribromure de bore suivi d'une salification.

Le groupe nitro du composé de formule (VII) est ensuite réduit par l'hydrogène, en présence d'un catalyseur tel que le palladium sur support de carbone, et également sous pression. Enfin l'acylation fournissant le composé final (I) peut s'effectuer de manière connue, à froid, par l'acide formique en présence d'anhydride acétique.

Lorsque l'on désire préparer un composé de formule I sous la forme d'un seul énantiomère, on introduit dans le processus une étape de séparation des énantiomères de l'amine primaire de formule V (dans ce cas les composés finals ne seront pas préparés par action d'une dialkylamine sur la cétone de formule (II), mais par alkylation directe de l'énantiomère séparé de l'amine primaire de formule V).

La résolution peut s'effectuer au moyen de la formation de sels diastéréoisomères à partir d'acides chiraux tels que l'acide tartrique, l'acide mandélique, l'acide camphosulfonique et, de préférence, l'acide dibenzoyltartrique. La salification se fait à partir d'un mélange d'énantiomères de l'amine primaire de formule (V) par exemple à partir du racémate obtenu à partir de la cétone (II), sous forme de base, dissous sans un solvant approprié, tel qu'un alcool, de préférence l'éthanol.

Les deux sels diastéréoisomères ainsi obtenus sont ensuite séparés par cristallisation fractionnée.

Après la séparation, ils peuvent être séparés de l'acide chiral, c'est à dire remis sous forme de base, puis sous forme de sel d'addition à un autre acide.

Un énantiomère de formule V peut également être obtenu à partir d'une base partiellement enrichie en cet énantiomère, provenant par exemple des eaux mères de recristallisation d'un mélange de sela diastéréoisomères. L'acide chiral utilisé pour la séparation d'un tel mélange enrichi est alors l'antipode de celui qui a permis d'obtenir le mélange enrichi.

Les exemples suivants illustrent la préparation de quelques composés de l'invention.

Les analyses et les spectres IR et RHN confirment la structure des composés.

EXEMPLE 1 Bromhydrate de dipropylamino-2 formylamino-5 hydroxy-6 tétrahydro-1,2,3,4 naphthalène.

a) Dipropylamino-2 méthoxy-6 tétrahydro-1,2,3,4 naphthalène. Dans 300 ml de benzène, sous atmosphère

d'azote, on introduit 20 g de méthoxy-6 tétrahydronaphthalénone-2, 20 ml de dipropylamine et 200 mg d'acide paratoluènesulfonique. On chauffe ensuite le mélange, devenu noir, au reflux pendant 12 heures, l'eau de condensation étant éliminée par entraînement azéotropique. Puis on concentre la solution qui contient le composé (IV), jusqu'à un volume d'environ 100 ml. Pour l'hydrogénation, on lui ajoute 150 ml d'éthanol, 300 mg de $PtO_2$ et on effectue une hydrogénation, sous une pression d'environ 0,3 MPa, jusqu'à cessation de l'absorption. Après élimination du catalyseur, on chasse les solvants sous pression réduite, et on reprend l'huile noire résiduelle par du toluène, pour l'extraire avec de l'acide chlorhydrique normal. Ensuite on neutralise la solution chlorhydrique par un alcali, on l'extrait par du toluène, on sèche la phase organique, on la filtre sur 200 mg d'alumine neutre, et on achève l'élution au moyen de chlorure de méthylène. Par concentration on obtient une huile pratiquement incolore.

b) Dipropylamino-2 méthoxy-6 nitro-5 et -7 tétrahydro-1,2,3,4 naphthalène.

Tout en refroidissant le mélange, on ajoute 21 g de dipro-pylamino-2 méthoxy-6 tétrahydronaphthalène à 60 ml d'acide trifluoroacétique. Ensuite, en maintenant la température à environ 0°C, on ajoute goutte à goutte 7 ml d'acide nitrique (d = 2,42). On agite encore 10 minutes, et on verse le tout dans de l'eau et on extrait l'insoluble par du chlrorure de méthylène; on agite la phase organique avec une solution de carbonate de potassium et on lave à l'eau.

Après séchage et évaporation, on soumet la gomme brune obtenue à une chromatographie sur colonne d'alumine neutre (800 g) en éluant avec du toluène. Le composé le moins polaire est l'isomère nitré en 5. On obtient 9 g de chacun des isomères. Le composé nitré en 5 fond à 198-200°C, le composé nitré en 7 fond à 158-160°C (sous formes de chlorhydrates).

c) Bromhydrate de dipropylamino-2 hydroxy-6 nitro-5 tétra-hydro-1,2,3,4 naphthalène.

On introduit 9 g de dipropylamino-2 méthoxy-6 nitro-5 tétrahydronaphthalène dans 100 ml d'acide bromhydrique à 48% et on chauffe au reflux pendant 2 heures. On chasse ensuite l'acide sous pression réduite, et on reprend le résidu trois fois avec de l'eau que l'on évapore pour éliminer toute trace d'acide.

Le solide obtenu, recristallisé dans l'eau, forme un monohydrate qui fond à 236°C (avec décomposition).

d) Bromhydrate de dipropylamino-2 amino-5 hydroxy-6 tétra-hydro-1,2,3,4 naphthalène.

On met en suspension 7 g de bromhydrate de dipropylamino-2 hydroxy-6 nitro-5 tétrahydronaphthalène dans 250 ml d'éthanol, et on effectue une hydrogénation à température ambiante, en présence d'l g de palladium sur charbon à 5%, sous une pression d'environ 0,3 MPa. Ensuite on évapore le solvant et on triture le résidu dans l'éther. Le produit obtenu fond à 215-218°C (avec décomposition).

e) Bromhydrate de dipropylamino-2 formylamino-5 hydroxy-6 tétrahydro-1,2,3,4 naphthalène.

A 2 ml d'acide formique à 98%, maintenus à 0°C, on ajoute en une fois 0,42 ml d'anhydride acétique et on laisse à 0°C pendant 15 minutes. Ensuite, toujours au bain de glace, on y introduit, à la spatule, 1,34 g de bromhydrate de dipropyl-amino-2 amino-5 hydroxy-6 tétrahydronaphthalène, et on agite le tout pendant une heure à 0°C.

Après addition d'éther et filtration du solide, on recristallise ce dernier dans le minimum d'éthanol. On obtient le composé qui fond à 199°C.

EXEMPLE 2 Bromhydrate de dipropylamino-2 formylamino-7 hydroxy-6 tétrahydro-1,2,3,4 naphthalène.

a) Dipropylamino-2 amino-7 méthoxy-6 tétrahydro-1,2,3,4 naphthalène.

On introduit 9 g de dipropylamino-2 méthoxy-6 nitro-7 tétrahydronaphthalène obtenus selon l'exemple 1b) dans 200 ml d'éthanol, ainsi que 1 g de Nickel de Raney. A température ambiante on effectue une hydrogénation sous pression jusqu'à cessation de l'absoprtion. Après évaporation du solvant il reste une huile qui se révèle assez oxydable à l'air.

b) Dibromhydrate de dipropylamino-2 amino-7 hydroxy-6 tétra-hydro-1,2,3,4 naphthalène.

On introduit le produit précédemment obtenu dans 100 ml d'acide bromhydrique à 48%, on chauffe au reflux pendant 10 heures puis on chasse l'acide sous pression réduite en l'éliminant ensuite complètement par entrainement avec un mélange toluène/éthanol, dans un évaporateur rotatif. Après recristallisation dans l'alcool isopropylique, le dibromhydrate fond à 205°C (avec décomposition).

c) Monohydrate de dipropylamino-2 amino-7 hydroxy-6 tétra-hydro-1,2,3,4 naphthalène.

A 3 g de dibromhydrate dissous dans 50 ml d'eau on ajoute 5 ml de résine Amberlite LA2 en solution dans 50 ml d'éther de pétrole. On agite pendant 15 minutes à température ambiante, on sépare la phase aqueuse et on l'évapore à sec pour isoler le monohydrate brut, sous forme amorphe et colorée, que l'on utilise tel quel pour la formylation.

d) Bromhydrate de dipropylamino-2 formylamino-7 hydroxy-6 tétrahydro-1,2,3,4 naphthalène.

A 3 ml d'acide formique à 98% maintenu à 0°C on ajoute 0,7 ml d'anhydride acétique et laisse le tout pendant 15 minutes à 0°C. On y ajoute ensuite 2,2 g de bromhydrate de dipropylamino-2 amino-7 hydroxy-6 tétrahydronaphthalène et on agite encore une heure à 0°C. Après addition de 50 ml d'éther et filtration du solide, on recristallise ce dernier dans un mélange 50/50 de méthanol/acétate d'éthyle. On obtient 1 g du produit final qui fond à 213°C (avec décomposition).

EXEMPLE 3 Enantiomères de bromhydrate de dipropylamino-2 formylamino-5 hydroxy-6 tétrahydro-1,2,3,4 naphthalène.

a) Amino-2 méthoxy-6 tétrahydro-1,2,3,4 naphthalène.

On chauffe au reflux pendant deux heures un mélange de 10 g de méthoxy-6 tétrahydronaphtalénone-2 dans 150 ml de benzène et de 5,9 ml de benzylamine avec 100 mg d'acide paratoluènesulfonique, sous atmosphère d'azote. L'eau de condensation est éliminée par entraînement azéotropique. On concentre le mélange à un

volume de 100 ml et on le soumet à une hydrogénation catalytique en présence de 100 mg de $PtO_2$, sous une pression de 0,3 MPa et à température ambiante. On élimine le catalyseur par filtration et on ajoute 4,7 ml d'acide chlorhydrique 12N au filtrat.

On procède alors à une seconde hydrogénation en présence de 1 g de palladium sur charbon, à environ 60°C, à une pression de l'ordre de 0,35 MPa.

On élimine le catalyseur, on chasse les solvants sous pression réduite et on recristallise le produit formé dans l'alcool isopropylique; il fond à 254°C.

On met le chlorhydrate sous forme de base de manière connue, par exemple en évaporant la phase organique d'un mélange de chlorure de méthylène, d'eau et de soude dans lequel on a introduit le chlorhydrate.

b) Séparation des énantiomères de l'amino-2 méthoxy-6 tétra-hydro-1,2,3,4 naphthalène.

A 5,5 g de l'amine (base) racémique obtenue comme indiqué ci-dessus, dissous dans 100 ml d'éthanol, on ajoute rapidement 6 g d'acide L(-)-dibenzoyltartrique dissous dans 100 ml d'éthanol. On concentre sous pression réduite le milieu hétérogène ainsi obtenu, on le reprend par de l'éther et, après filtration et séchage, on receuille II g de sel contenant 1/2 mole d'acide L(-)-dibenzoyltartrique. On recristallise deux fois ce dernier dans de l'éthanol contenant 30% d'eau. Le produit obtenu fond à 220-221°C. En le mettant sous forme de chlorhydrate, on obtient un sel optiquement actif fondant a 254°C (avec décomposition) et de pouvoir rotatoire

$$[\alpha]_D^{25} = -73° \quad (c=1,\ MeOH)$$

Pour isoler l'autre énantiomère, on concentre les eaux mères éthanoliques de recristallisation du L(-)-dibenzoyltartrate, on repasse à la forme base de l'amine, après l'avoir extraite, on en précipite le D(+)-dibenzoyltartrate par addition de l'acide correspondant. Après recristallisation, le D(+)-dibenzoyltartrate fond à 220-221°C. Par formation du chlorhydrate on obtient un sel optiquement actif de pouvoir rotatoire

$$[\alpha]_D^{25} = +73° \quad (c=1,\ MeOH)$$

c) Chlorhydrate de (-)-dipropylamino-2 méthoxy-6 tétrahy-dro-1,2,3,4 naphthalène.

A 1,9 g de chlorhydrate lévogyre obtenu comme indiqué ci-dessus, en suspension dans 20 ml de benzène, on ajoute 15 ml de solution saturée de carbonate de potassium puis 9 ml d'iodopropane. On agite le mélange à la température de reflux pendant 72 heures. Ensuite on dilue la phase organique avec de l'éther, on forme le chlorhydrate par addition d'éther chlorhydrique et on l'isole de manière habituelle. Il fond à 154°C et présente un pouvoir rotatoire

$$[\alpha]_D^{25} = -2,2°, \quad (c=1,\ MeOH).$$

d) Bromhydrate de (-)-dipropylamino-2 formylamino-5 hydro-xy-6 tétrahydronaphthalène.

On met en oeuvre le mode opératoire de l'exemple 1b), c), d), et e), en utilisant l'énantiomère préparé comme indiqué ci-dessus, au lieu du mélange racémique.

Les chlorhydrates des composés intermédiaires nitrés en 5 et 7 ont des pouvoirs rotatoires respectifs de

$$[\alpha]_D^{25} = -140° \quad et \quad [\alpha]_D^{25} = -76° \quad (c=1,\ MeOH).$$

Le bromhydrate intermédiaire réduit ($NH_2$ en 5) a un pouvoir rotatoire de -32° (c=1,MeOH).

Le bromhydrate du composé final, énantiomère lévogyre du composé préparé selon l'exemple 1, a un pouvoir rotatoire de

$$[\alpha]_D^{25} = -48° \quad (c=1,\ MeOH).$$

Les pouvoirs rotstoires des énantiomères dextrogyres sont, en valeur absolue, égaux à ceux des énantiomères, aux erreurs de mesures près.

Les composés de l'invention sont illustrés dans le tableau 1 ci-après, par l'indication de leur structure et de leurs propriétés physiques.

Le tableau II illustre les structures et propriétés physiques de quelques composés intermédiaires de formule VI et VII.

**TABLEAU I**

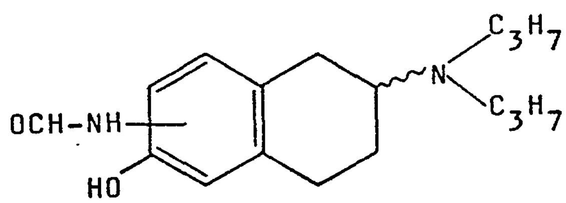
(I)

| Exemple | Position de -NH-CHO | F(°C) | Sel (*) |
|---|---|---|---|
| 1e) | 5 | 199 | HBr |
| 2d) | 7 | 213 | HBr |
| 3 | 5 | $[\alpha]_D^{25} = -48°$ | HBr |
| 4 | 7 | $[\alpha]_D^{25} = -48°$ | HBr |

(*) HBr: bromhydrate

TABLEAU II

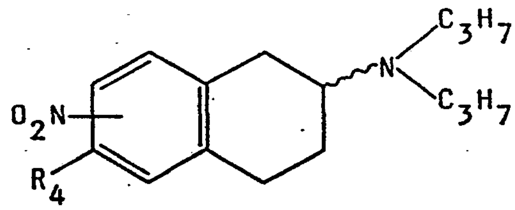

| Position de $-NO_2$ | $R_4$ | F(°C) | Sel (*) |
|---|---|---|---|
| 5 | $-OCH_3$ | 198-200 | HCl |
| 7 | $-OCH_3$ | 158-160 | HCl |
| 5 | -OH | 236 | HBr |
| 5 | $-OCH_3$ | $[\alpha]_D^{25} = -140°$ | HCl |
| 7 | $-OCH_3$ | $[\alpha]_D^{25} = -76°$ | HCl |

(*) HCl: chlorhydrate
HBr: bromhydrate

Les composés de formule I ont été soumis à des essais pharmacologiques qui ont mis en évidence leur activité antihypertensive et antiparkinsonnienne.

On a constaté ainsi que sur le rat ou le chien anesthésié au pentobarbital sodique, la tension atérielle diminue nettement après des injections de 10 à 100 µg/kg par voie intraveineuse.

Par ailleurs, administrés au chien par voie intraveineuse ou intraduodénale, les composés de l'invention inhibent les réponses aux stimulations électriques de la membrane nictitante et du coeur, leur activité étant dans ce cas environ 20 fois supérieure à celle d'un dopaminergique connu, la N,N-dipropyldopamine.

Les effets des composés de l'invention sont bloqués par l'action du sulpiride, un antagoniste connu de la dopamine.

L'activité antiparkinsonienne des composés de l'invention a été mise en évidence par le test de l'antagonisme vis-à-vis de la catalepsie induite par le halopéridol.

On sait qu'un certain nombre de neuroleptiques induisent chez le rat un état cataleptique considéré comme le reflet d'un blocage des récepteurs dopaminergiques au niveau du système extrapyramidal: c'est le cas du halopéridol, dont l'effet cataleptigène est antagonisé par les agonistes dopaminergiques tels que l'apomorphine ou l'amphétamine, par les antidépresseurs tricycliques et par les anticholinergiques. La méthode utilisée ici est une modification de celle décrite par Tedeschi et al., Arch. Int. Pharmacodyn. (1959), 122, 129.

Les rats recoivent le halopéridol par voie intrapéritonéale à la dose de 1 mg/kg sous un volume de 1 ml/IOO g de poids corporel.

Trente minutes après cette injection, les produits à étudier sont administrés, à 6 rats par dose, et à raison de 3 doses par produit étudié.

On évalue la catalepsie des animaux trente minutes (voie i.p.) ou 60 minutes (per os) après l'administration du produit à étudier, puis toutes les 30 minutes, pendant 3 heures (voie intrapéritonéale) ou 4 heures (per os). Pour cela on place chaque animal dans une position telle que chaque patte repose sur un "bouchon" de 25 mm de hauteur et 12 mm de diamètre.

L'animal est considéré comme cataleptique s'il se maintient dans cette position pendant au moins 10 secondes.

Pour chaque dose et chaque produit on calcule le pourcentage moyen d'animaux cataleptiques, puis le pourcentage de diminution par rapport aux animaux témoins.

La DA5O, dose qui antagonise de 50% l'effet cataleptique du halopéridol, est déterminée graphiquement.

On a ainsi trouvé que la DA5O des composés de l'invention est de l'ordre de 7 mg/kg, tant par voie intrapéritonéale que par voie orale.

Enfin leur toxicité aigòe chez la Souris est de l'ordre de 90 mg/kg par voie intraveineuse et de 150 mg/kg par voie orale.

Compte tenu de leurs propriétés, les composés de l'invention sont utilisables pour le traitement de maladies cardiovasculaires, en particulier pour le traitement de l'hypertension, ainsi que pour le traitement de la maladie de Parkinson. Ils peuvent être administrés à raison de doses journalières de 1 à 500 mg par unités de prise contenant par exemple de 1 à 100 mg de substance active, par voie orale, rectale ou parentérale.

**Revendications**

pour les Etats contractants: BE, CH, DE, FR, GB, IT, LI, LU, NL, SE

1. Composés sous forme de racémates ou d'énantiomères répondant à la formule générale 1

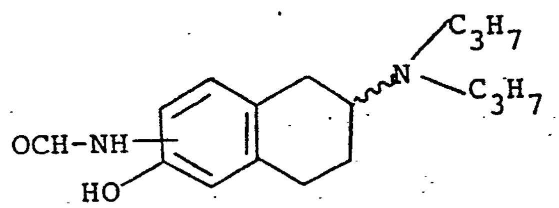

(I)

dans laquelle le groupe -NH-CHO est en ortho du groupe -OH, ainsi que leurs sels d'addition à des acides acceptables en pharmacologie.

2. Le dipropylamino-2 formylamino-5 hydroxy-6 tétrahydro -1,2,3,4 naphthalène et ses sels d'addition à des acides acceptables en pharmacologie.

3. Le dipropylamino-2 hydroxy-6 formylamino-7 tétrahydro -1,2,3,4 naphthalène et ses sels d'addition à des acides acceptables en pharmacologie.

4. Procédé de préparation des composés selon la revendication 1, caractérisé en ce qu'on fait réagir la méthoxy-6 tétrahydro-1,2,3,4 naphthalénone-2 avec la benzylamine ou la dipropylamine, puis on effectue une hydrogénation du composé obtenu, répondant à la formule IV

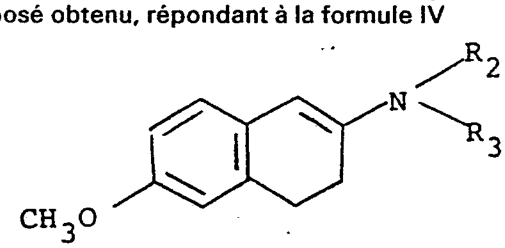

(IV)

dans laquelle $-NR_2R_3$ est le groupe amino ou dipropylamino, pour obtenir le composé de formule V

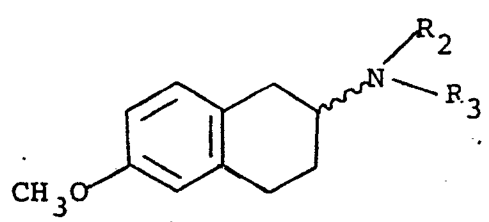

(V)

dont, si $-NR_2R_3$ représente le groupe amino primaire, on sépare éventuellement les énantiomères puis on dipropyle ledit groupe amino primaire de l'un des énantiomères ou du racémate, puis on soumet le composé (V) ainsi obtenu à une nitration puis à une séparation d'isomères pour obtenir un composé de formule VI

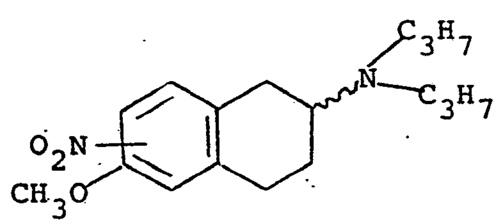

(VI)

dans laquelle le groupe $-NO_2$ est en ortho du groupe $-OCH_3$, puis on effectue une déméthylation, d'où résulte un composé de formule VII

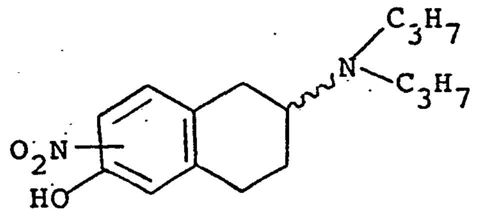

(VII)

puis on soumet le composé de formule VII à une réduction catalytique par l'hydrogène pour obtenir un composé de formule VIII

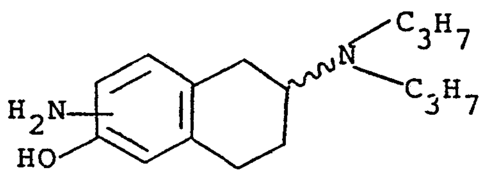

(VIII)

et enfin on effectue une N-formylation pour obtenir un composé de formule 1 tel que défini dans la revendication 1.

5. Procédé selon la revendication 4, caractérisé en ce qu'on effectue la séparation des énantiomères de l'amine primaire de formule V en préparant les diastéréoisomères des sels qu'elle forme avec les antipodes de l'acide dibenzoyltartrique et en séparant lesdits diastéréoisomères.

6. Composés nécessaires comme intermédiaires dans le procédé de préparation selon la revendication 4, caractérisés en ce qu'ils répondent à la formule générale

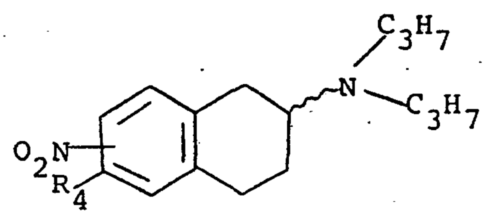

dans laquelle $R_4$ représente un groupe hydroxyle ou méthoxyle, le groupe $-NO_2$ étant en ortho du groupe $R_4$.

7. Médicament contenant un composé selon l'une quelconque des revendications 1 à 3.

8. Composition pharmaceutique, caractérisée en ce qu'elle contient à titre de substance active un composé selon l'une quelconque des revendications 1 à 3, associé à un véhicule acceptable en pharmacologie.

**Revendications**

pour l'Etat contractant: AT

1. Procédé de préparation de composés répondant à la formule générale I

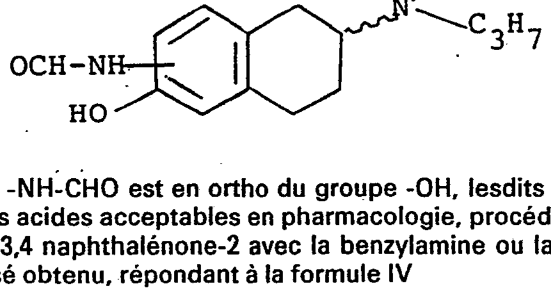

(I)

dans laquelle le groupe -NH-CHO est en ortho du groupe -OH, lesdits composés étant sous forme de bases ou de sels d'addition à des acides acceptables en pharmacologie, procédé caractérisé en ce qu'on fait réagir la méthoxy-6 tétrahydro-1,2,3,4 naphthalénone-2 avec la benzylamine ou la dipropylamine, puis on effectue une hydrogénation du composé obtenu, répondant à la formule IV

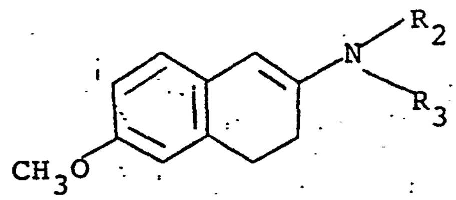

(IV)

dans laquelle $-NR_2R_3$ est le groupe amino ou dipropylamino, pour obtenir le composé de formule V

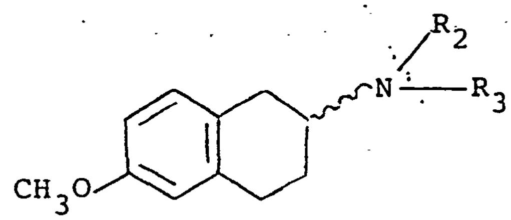

(V)

dont, si $-NR_2R_3$ représente le groupe amino primaire, on sépare éventuellement les énantiomères puis on dipropyle ledit groupe amino primaire de l'un des énantiomères ou du racémate, puis on soumet le composé (V) ainsi obtenu à une nitration puis à une séparation d'isomères pour obtenir un composé de formule VI

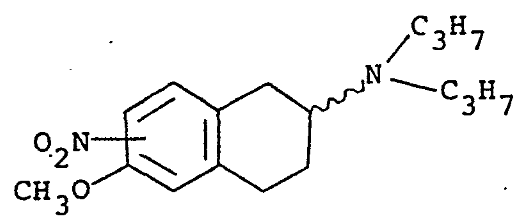

(VI)

dans laquelle le groupe $-NO_2$ est en ortho du groupe $-OCH_3$, puis on effectue une déméthylation, d'où résulte un composé de formule VII

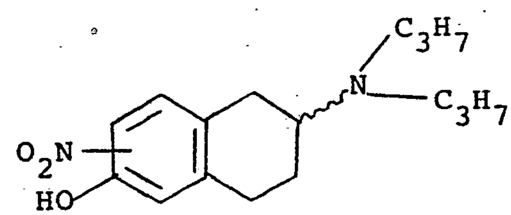

(VII)

puis on soumet le composé de formule VII à une réduction catalytique par l'hydrogène pour obtenir un composé de formule VIII

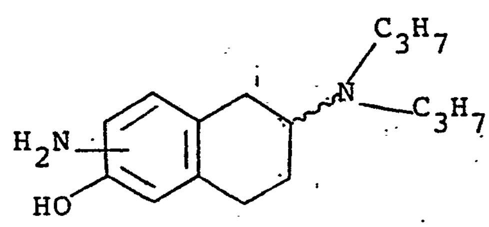

(VIII)

et enfin on effectue une N-formylation pour obtenir un composé de formule 1 tel que défini ci-dessus.

2. Procédé selon la revendication 1, caractérisé en ce qu'on effectue la séparation des énantiommères de l'amine primaire de formule V en préparant les diastéréoisomères des sels qu'elle forme avec les antipodes de l'acide dibenzoyltartrique et en séparant lesdits diastéréoisomères.

**Patentansprüche**

für die Vertragsstaaten BE, CH,DE, FR, GB, IT, LI, LU, NL, SE

1. Verbindungen in Form ihrer Racemate oder Enantiomeren, die der allgemeinen Formel I

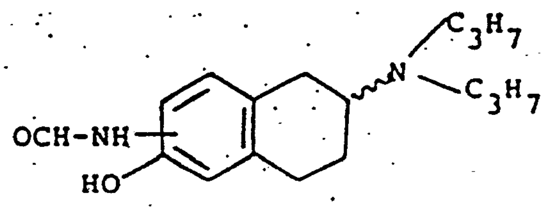

(I)

entsprechen, in welcher die Gruppe -NH-CHO in ortho-Stellung zur OH-Gruppe steht, sowie deren Additionssalze mit pharmakologisch verwendbaren Säuren.

2. 2-Dipropylamino -5-formylamino-6-hydroxy-1,2,3,4-tetrahydronaphthalin und seine Additionssalze mit pharmakologisch verwendbaren Säuren.

3. 2-Dipropylamino-6-hydroxy-7-formylamino-1,2,3,4-tetrahydronaphthalin und seine Additionssalze mit pharmakologisch verwendbaren Säuren.

4. Verfahren zur Herstellung der Verbindungen nach Anspruch 1, dadurch gekennzeichnet, daß man 6-Methoxy-1,2,3,4-tetrahydronaphthalin-2-on mit Benzylamin oder Dipropylamin reagieren läßt, dann eine Hydrierung der erhaltenen Verbindung der Formel IV

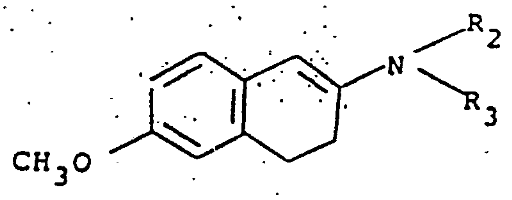

(IV)

vornimmt, in welcher $-NR_2R_3$ für die Animo-oder Dipropylaminogruppe steht, um die Verbindung der Formel V

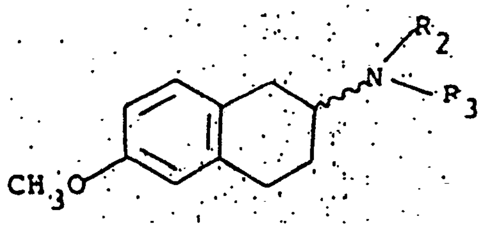

(V)

zu gewinnen,von der man, wenn $-NR_2R_3-$ die primäre Aminogruppe darstellt. gegebenenfalls die Enantiomeren abtrennt, dann die genannte primäre Aminogruppe des einen der Enantiomeren oder des Racemats dipropyliert dann die so erhaltene Verbindung (V) nitiert und dann die Isomeren auftrennt, um eine Verbindung der Formel VI

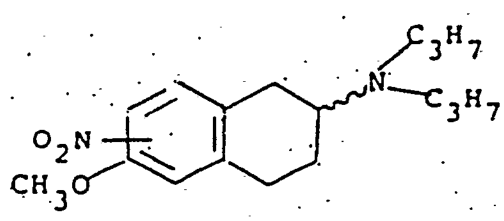

(VI)

zu erhalten, in welcher die $NO_2$-Gruppe in ortho-Stellung zu der $CH_3O$-gruppe steht, dann eine Demethylierung vornimmt, aus welcher eine Verbindung der Formel VII

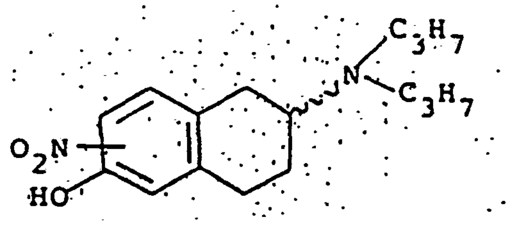

(VII)

stammt, worauf man die Verbindung der Formel VII einer katalytischen Reduktion mit Wasserstoff unterzieht um eine Verbindung der Formel VIII

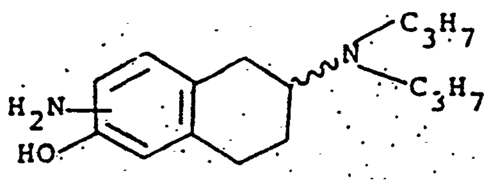

(VIII)

zu erhalten, und daß man schließlich eine N-Formylierung vornimmt, um eine Verbindung der im Anspruch 1 definierten Formel I zu erhalten.

5. Verfahren nach Anspruch 4, dadurch gekennzeichnet, daß man die Trennung der Enantiomeren des primären Amins der Formel V dadurch bewirkt, daß man die Diastereoisomeren der Salze, die die Verbindung mit den Antipoden der Dibenzoylweinsäure bildet, herstellt und diese Diastereoisomeren trennt.

6. Verbindungen, die als Zwischenprodukte in dem Herstellungsverfahren nach Anspruch 4 nötig sind, dadurch gekennzeichnet, daß sie der allgemeinen Formel

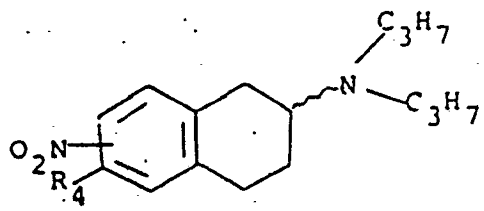

entsprechen, in welcher $R_4$ eine Hydroxygruppe oder eine Methoxygruppe bedeutet, wobei die $NO_2$-Gruppe in ortho-Stellung zu der Gruppe $R_4$ steht.

7. Heilmittel enthaltend eine Verbindung nach irgendeinem der Ansprüche 1 bis 3.

8. Pharmazeutische Zusammensetzung, dadurch gekennzeichnet, daß sie als Wirkstoff eine Verbindung nach irgendeinem der Ansprüche 1 bis 3 in Kombination mit einem pharmakologisch verwendbaren Träger enthält.

**Patentansprüche**

für den Vertragsstaat Österreich

1. Verfahren zur Herstellung von Verbindungen der allgemeinen Formel I

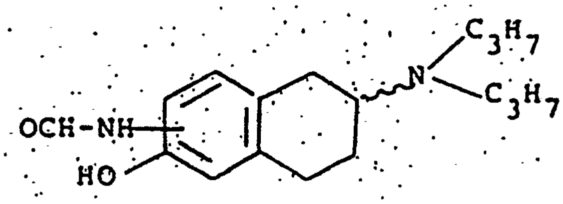

(I)

in welcher die Gruppe -NH-CHO in ortho-Stellung zu OH-Gruppe steht, wobei die Verbindungen in Form der Basen oder der Additionssalze mit pharmakologisch verwendbaren Säuren vorliegen, dadurch gekennzeichnet, daß man 6-Methoxy-1,2,3,4-tetrahydronaphthalin-2-on mit Benzylamin oder Dipropylamin reagieren läßt, daß man eine Hydrierung der erhaltenen Verbindung der Formel IV

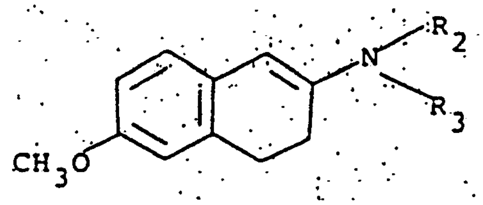

(IV)

in welcher - $NR_2R_3$ für die Amino- oder Dipropylaminogruppe steht, vornimmt, um die Verbindung der Formel V

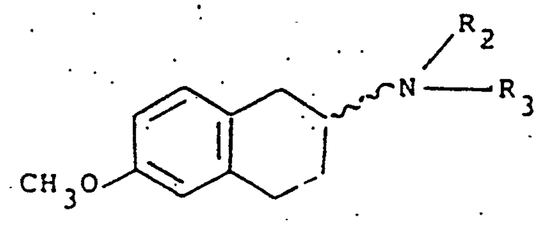

(V)

zu erhalten, von welcher man, wenn - $NR_2R_3$ für die primäre Aminogruppe steht, gegebenenfalls die Enantiomeren abtrennt, dann die genannte primäre Aminogruppe des einen der Enantiomeren oder des Racemats dipropyliert, dann die so erhaltene Verbindung-(V) nitriert, dann die Isomeren trennt, um eine Verbindung der Formel VI

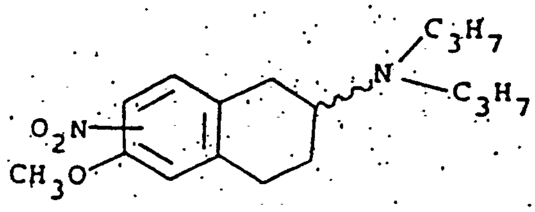

(VI)

zu elhalten, in welcher die $NO_2$-Gruppe in ortho-Stellung zur $CH_3O$-Gruppe steht, worauf man eine Demethylierung vornimmt, die eine Verbindung der Formel VII

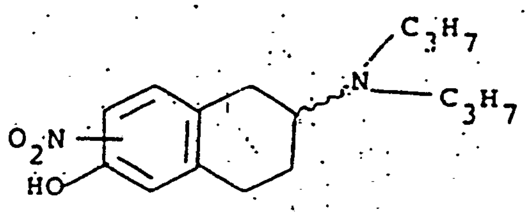

(VII)

ergibt, worauf man die Verbindung der Formel VII einer katalytischen Reduktion mit Wasserstoff unterwirft, um eine Verbindung der Formel VIII

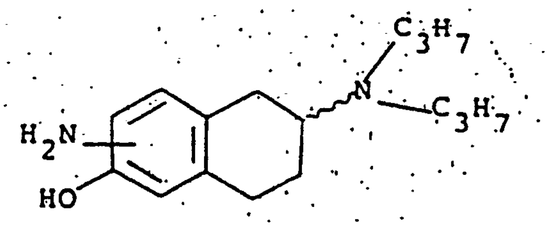

(VIII)

zu erhalten, und daß man schließlich eine N-Formylierung vornimmt, um eine Verbindung der oben definierten Formel I zu erhalten.

2. Verfahren nach Anspruch 1, dadurch gekennzeichnet, daß man die Auftrennung der Enantiomeren des primären Amins der Formel V vornimmt, indem man-die Diastereoisomeren der Salze, die die Verbindung mit den Antipoden der Dibenzoylweinsäure bildet, herstellt und diese Diastereoisomeren trennt.

**Claims**

for the contracting States: BE, CH, DE, FR, GB, IT, LI, LU, NL, SE.

1. Compounds in form of racemates or enantiomers responding to general formula I

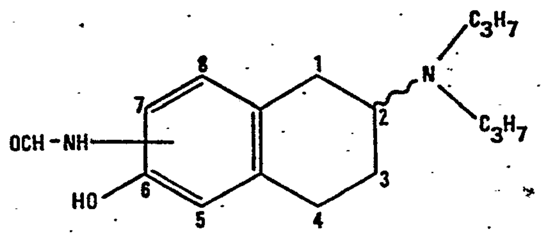

(I).

wherein the -NH-CHO group is located in ortho of the -OH group, and their pharmacologically acceptable acid addition salts.

2. 2-Dipropylamino-5-formylamino-6-hydroxy-1, 2, 3, 4-tetra-hydro-naphthalene, and its pharmacologically acceptable acid addition salts.

3. 2-Dipropylamino-6-hydroxy-7-formylamino-1,2,3,4-tetra-hydro-naphthalene, and its pharmacologically acceptable acid addition salts.

4. A process for the preparation of the compounds of claim 1, characterized in that 6-methoxy-1,2,3,4-naphthalen-2-one is reacted with benzylamine or dipropylamine, then the compound obtained, of formula IV

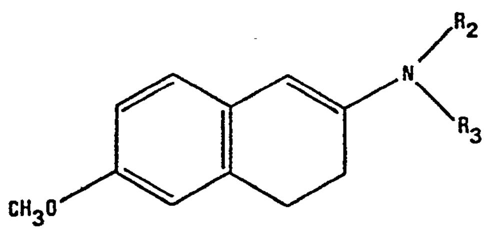

(IV)

wherein $-NR_2R_3$ is an amino or dipropylamino group, is hydrogenated so as to produce the compound of formula V

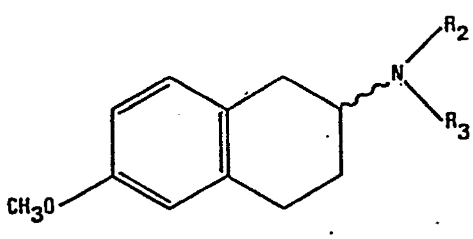

(V)

from which, if $-NR_2R_3$ denotes the primary amino group the enantiomers are optionally separated, then said primary amino group of one of the enantiomers or of the racemate is dipropylated, then the compound (V) obtained is subjected to a nitration followed by separation of isomers to produce a compound of formula VI

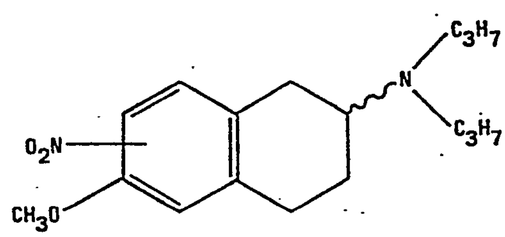

(VI)

wherein the $-NO_2$ group is located in ortho of the $-OCH_3$ group, then a demethylation is carried out, producing a compound of formula VII

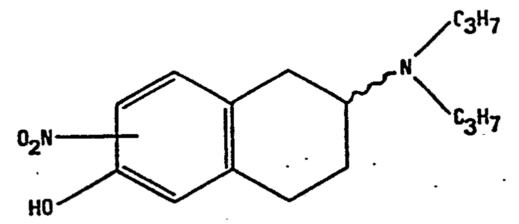

(VII)

then the compound of formula VII is subjected to a catalytic reduction with hydrogen so as to produce a compound of formula VIII

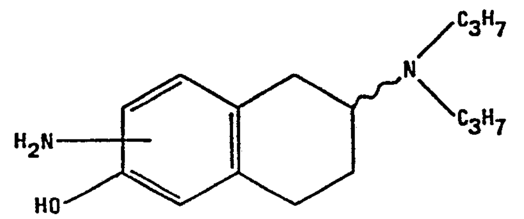

(VIII)

and finally a N-formylation is carried out so as to produce a compound of formula I as specified in claim 1.

5. A process according to claim 4, characterized in that the separation of the enantiomers of the primary amine of formula V is carried out by preparing the diastereoisomers of the salts formed with the antipodes of dibenzoyltartaric acid, and by separating said diastereoisomers.

6. Compounds necessary as intermediates in the process of preparation according to claim 4, characterized in that they respond to the general formula

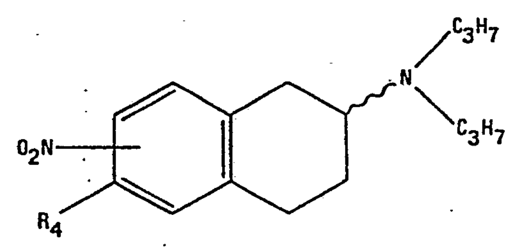

wherein $R_4$ denotes a hydroxyl or methoxyl group, the $-NO_2$ group being located in ortho of the $R_4$ group.

7. A drug containg a compound according to anyone of claims 1 to 3.

8. A pharmaceutical composition, characterized in that it contains, as an active substance, a compound according to anyone of claims 1 to 3, in combination with a pharmacologically acceptable carrier.

**Claims**

for the contracting State: AT.

1. A process for the preparation of compounds responding to general formula I

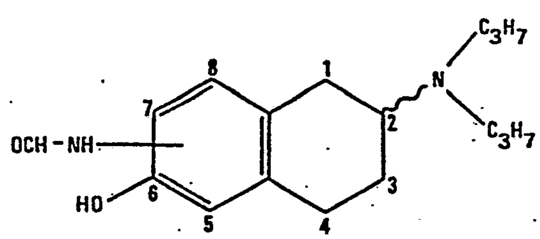

(I)

wherein the -NH-CHO group is located in ortho of the -OH group, said compounds being in form of bases or addition salts with pharmacologically acceptable acids, process characterized in that 6-methoxy-1,2,3,4-naphthalen-2-one is reacted with benzylamine or dipropylamine, then the compound obtained, of formula IV

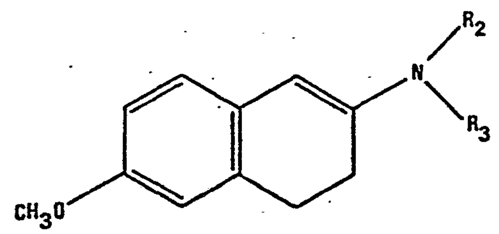

(IV)

wherein $-NR_2R_3$ is an amino or dipropylamino group, is hydrogenated so as to produce the compound of formula V

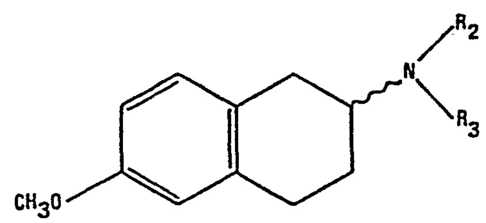

(V)

from which, if $-NR_2R_3$ denotes the primary amino group the enantiomers are optionally separated, then said primary amino group of one of the enantiomers or of the racemate is dipropylated, then the compound (V) obtained is subjected to a nitration followed by separation of isomers to produce a compound of formula VI

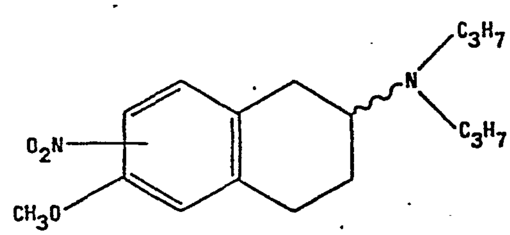

(VI)

wherein the $-NO_2$ group is located in ortho of the $-OCH_3$ group, then a demethylation is carried out, producing a compound of formula VII

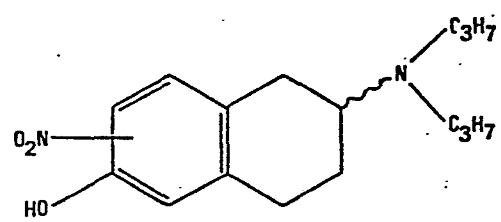

(VII)

then the compound of formula VII is subjected to a catalytic reduction with hydrogen so as to produce a compound of formula VIII

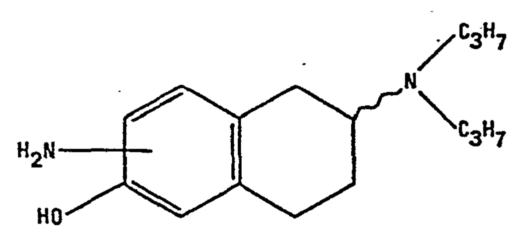

(VIII)

and finally a N-formylation is carried out so as to produce a compound of formula I as specified above.

2. A process according to claim 1, characterized in that the separation of the enantiomers of the primary amine of formula V is carried out by preparing the diastereoisomers of the salts formed with the antipodes of dibenzoyltartaric acid, and by separating said diastereoisomers.